# EUROPEAN PATENT APPLICATION

(11) **EP 3 284 388 A1**
(43) Date of publication of application: **21.02.2018**
(21) Application number: 16779996.4
(22) Date of filing: 11.04.2016
(51) Int. Cl.: A61B 1/00, G01N 21/17

(54) **OPTICAL PROBE**

(30) Priority: 16.04.2015 JP 2015083896
(71) Applicant: Sumitomo Electric Industries, Ltd., Osaka-shi, Osaka 541-0041 (JP)
(72) Inventor: SASAKI, Dai, Yokohama-shi Kanagawa 244-8588 (JP); TAMEKUNI, Yoshikyo, Yokohama-shi Kanagawa 244-8588 (JP)
(74) Representative: Hoffmann Eitle
(86) International application number: PCT/JP2016/061649
(87) International publication number: WO 2016/167205

(57) **Abstract**

An optical probe that has an end having a reduced diameter is provided. An optical probe has a proximal end to be connected to a measurement unit of an OCT device and a distal end from which observation light exits and includes an optical fiber, an optical connector that is connected to the OCT device, a Grin lens corresponding to a light collection optical system and a light deflection optical system that are optically connected to the optical fiber at the distal end, a needle that rotatably accommodates the optical fiber and the Grin lens, a handpiece that rotatably accommodates a part of the optical fiber between the proximal end and the distal end and that holds the needle, and a support tube that is secured to the optical connector on a proximal end side and that is rotatably accommodated in the handpiece on a distal end side. An outer diameter of the needle is less than an outer diameter of the support tube.

## Description

### Technical Field

The present invention relates to an optical probe used for optical coherence tomography (OCT).

### Background Art

Optical coherence tomography (OCT) has been known as a method of measuring the sectional structure of an object such as an organism. During OCT measurement, an optical probe is inserted near the object, and observation light is emitted from the optical probe. The observation light reflected back from the object is captured by the optical probe. In JP2013-202295A (PTL 1), an optical probe suitable for OCT measurement of an object, for example, a body cavity such as a blood vessel is disclosed. According to the technique disclosed in PTL 1, an optical fiber, a light-collection light-deflection optical system, and a support tube through which a rotational torque is transmitted are accommodated in a jacket tube. Accordingly, there is a limit to reduction in the diameter of an end of the optical probe.

### Summary of Invention

### Technical Problem

An object of the present invention is to provide an optical probe that has an end having a reduced diameter and that is to be inserted into, for example, an organism.

### Solution to Problem

An optical probe including an optical fiber, an optical connector, a light collection optical system, a light deflection optical system, a needle, a handpiece, and a support tube is provided to achieve the object. Observation light is transmitted through the optical fiber between a proximal end to be connected to a measurement unit of an OCT device and a distal end from which the observation light exits. The optical connector is connected to the optical fiber at the proximal end and is connected to the measurement unit. The light collection optical system is optically connected to the optical fiber at the distal end and collects the observation light that exits from the optical fiber. The light deflection optical system is optically connected to the light collection optical system at the distal end and deflects the observation light that exits from the optical fiber. The needle is formed of a material through which the observation light is transmissible and rotatably accommodates the optical fiber, the light collection optical system, and the light deflection optical system at the distal end. The handpiece has a through-hole, rotatably accommodates a part of the optical fiber between the proximal end and the distal end, and holds the needle. The support tube is secured to the optical connector on a proximal end side and is rotatably accommodated in the handpiece on a distal end side. An outer diameter of the needle is less than an outer diameter of the support tube.

The through-hole of the optical probe according to the present invention may include a second section that rotatably accommodates the optical fiber and the support tube, a third section that rotatably accommodates the optical fiber, and a fourth section that secures the needle and that rotatably accommodates the optical fiber, and a relationship of
an inner diameter of the second section > an inner diameter of the fourth section
> an inner diameter of the third section
may hold. In this case, the third section may be formed of a material having a coefficient of friction less than a coefficient of friction of the second section and the fourth section.

The optical probe according to the present invention may further include a metallic tube that is secured inside the needle and that rotatably accommodates the optical fiber, the light collection optical system, and the light deflection optical system. In this case, the metallic tube may have, at a location in a circumferential direction, a slit that causes the observation light deflected by the light deflection optical system to exit from the distal end. Surface roughness of an inner surface of the metallic tube that rotatably accommodates the optical fiber, the light collection optical system, and the light deflection optical system is preferably less than surface roughness of an outer surface thereof that is a surface secured inside the needle. The needle may be secured to the fourth section with an adhesive, and an end portion of the metallic tube on the proximal end side may be located nearer than an end portion of the needle on the proximal end side to the distal end.

The optical fiber of the optical probe according to the present invention may include, in the needle, a coating and a glass fiber around which the coating is removed, and an outer circumference of the light deflection optical system, the light collection optical system, and the glass fiber may be covered by a molded portion through which the observation light is transmissible. In this case, the outer edge of the molded portion may be located inside the outer edge of the coating when viewed in a direction of an optical axis of the optical fiber.

The molded portion of the optical probe according to the present invention may include, on a part extending in an optical axis of the optical fiber, a protrusion that protrudes from an outer circumference of the molded portion toward the needle. In this case, the protrusion preferably includes a plurality of protrusions that are arranged in a circumferential direction of the optical fiber and that are equal in height from the molded portion.

### Advantageous Effects of Invention

According to the present invention, an optical probe that has an end having a reduced diameter and that is to be inserted into, for example, an organism can be provided.

### Brief Description of Drawings

Figure 1 is a schematic view of an OCT device including an optical probe according to an embodiment of the present invention.
Figure 2 at a (a) region illustrates the YZ section of the distal end of the optical probe in Figure 1. Figure 2 at a (b) region illustrates the front of a glass fiber, a coating, a molded portion, and a Grin lens included in the distal end of the optical probe in Figure 1 viewed from the Z-direction.
Figure 3 is a perspective view of the YZ section of the distal end of the optical probe in Figure 1.
Figure 4 schematically illustrates the functions of the optical probe in Figure 1.
Figure 5 illustrates the YZ section of an exemplary handpiece of the optical probe in Figure 1 on the distal end side.
Figure 6 illustrates the YZ section of another exemplary handpiece of the optical probe in Figure 1 on the distal end side.
Figure 7 illustrates the XY section of the distal end of an optical probe according to a modification to the embodiment.

### Description of Embodiments

A specific example of an optical probe according to an embodiment of the present invention will hereinafter be described with reference to the drawings. It is intended that the present invention is not limited to the embodiment, is shown by the scope of claims, and includes all modifications having the same content and range as the scope of claims. In the following description of the drawings, identical components are designated by the same reference numbers, and a duplicated description is omitted.

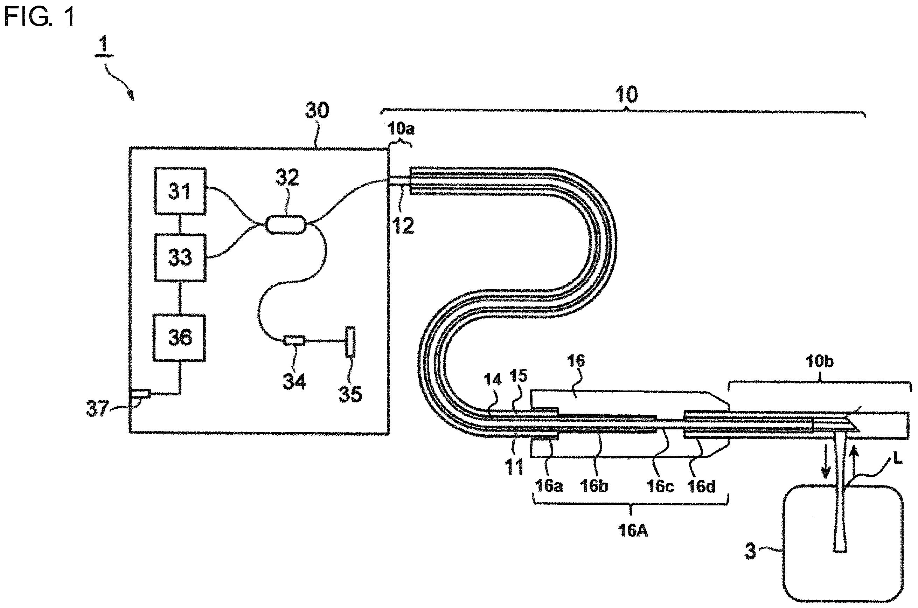

Figure 1 is a schematic view of an OCT device 1 including an optical probe 10 according to the embodiment of the present invention. The OCT device 1 includes the optical probe 10 and a measurement unit 30 and captures an optical coherence tomography image of an object 3. The optical probe 10 has a proximal end 10a and a distal end 10b and includes a handpiece 16 therebetween. An optical fiber 11 extends from the proximal end 10a to the distal end 10b and is inserted through a through-hole 16A of the handpiece 16. The distal end 10b of the optical probe 10 can be inserted into an organism that is an object to be observed while the handpiece 16 is held such that the tip of the distal end 10b is located near a portion of the object to be observed.

The measurement unit 30 includes a light source 31, a bifurcated portion 32, a detector 33, a terminal 34, a reflecting mirror 35, an analyzer 36, and an output port 37. Light emitted from the light source 31 is bifurcated into observation light and reference light at the bifurcated portion 32. The observation light reaches the proximal end 10a of the optical probe 10, propagates through the optical fiber 11, and is emitted from the distal end 10b to the object 3.

Back-reflection light resulted from the radiation of the observation light to the object 3 is incident again on the optical fiber 11 from the distal end 10b and enters the bifurcated portion 32 from the proximal end 10a. The reference light exits from the terminal 34 toward the reflecting mirror 35, is incident again on the terminal 34, and enters the bifurcated portion 32. The observation light and the reference light incident on the bifurcated portion 32 are combined and interfere with each other at the bifurcated portion 32, and interference light is detected by the detector 33. The spectrum of the interference light is analyzed by the analyzer 36, the distribution of back-reflection efficiency at points on the internal cross-section of the object 3 is calculated. A tomographic image of the object 3 is calculated on the basis of the calculation result, and an image signal is outputted from the output port 37.

Strictly speaking, mechanisms that cause the observation light to return again to the distal end 10b via the object 3 include reflection, refraction, and scattering. However, a difference between these is not essential for the present invention. Accordingly, in the description, a general term of these is referred to as back reflection for simplification.

The optical fiber 11 includes an optical connector 12 near the proximal end 10a and is optically connected to the measurement unit 30 with the optical connector 12 interposed therebetween. The OCT device 1 rotates the optical connector 12 to rotate the optical fiber 11 and scans the observation light in the circumferential direction to capture the optical coherence tomography image of the object 3 within a predetermined range.

The optical probe 10 includes a support tube 14 that covers the outer circumference of the optical fiber 11 and a jacket tube 15 that covers the outer circumference of the support tube 14 nearer than the handpiece 16 to the proximal end 10a. The optical fiber 11 and the support tube 14 are secured to the optical connector 12 and are rotatable with respect to the jacket tube 15.

The support tube 14 is a metallic hollow member and may be a thin tubular pipe member or may be formed in a tube whose flexibility is adjusted in a manner in which metallic fibers are twisted. The inner diameter of the support tube 14 is, for example, 0.4 to 0.6 mm, and a single mode optical fiber that has an outer diameter of 0.25 mm can be inserted through the support tube 14. The thickness of the support tube 14 is preferably about 0.3 mm to 0.7 mm so that the rotational torque of the optical connector 12 can be efficiently transmitted to the distal end 10b. Accordingly, the outer diameter of the support tube is about 1 to 2 mm.

The handpiece 16 has the through-hole 16A through which the optical fiber 11 is inserted. The through-hole 16A includes a first section 16a, a second section 16b, a third section 16c, and a fourth section 16d in this order in the direction from the proximal end 10a to the distal end 10b. The first section 16a is a section to which the jacket tube 15 is secured. The second section 16b rotatably accommodates the optical fiber 11 and the support tube 14. The third section 16c rotatably accommodates the optical fiber 11. The fourth section 16d secures the distal end 10b (a metallic tube 17 and a needle 18 described later) and rotatably accommodates the optical fiber 11. The detail of the structure and function of the second section 16b to the fourth section 16d will be described later.

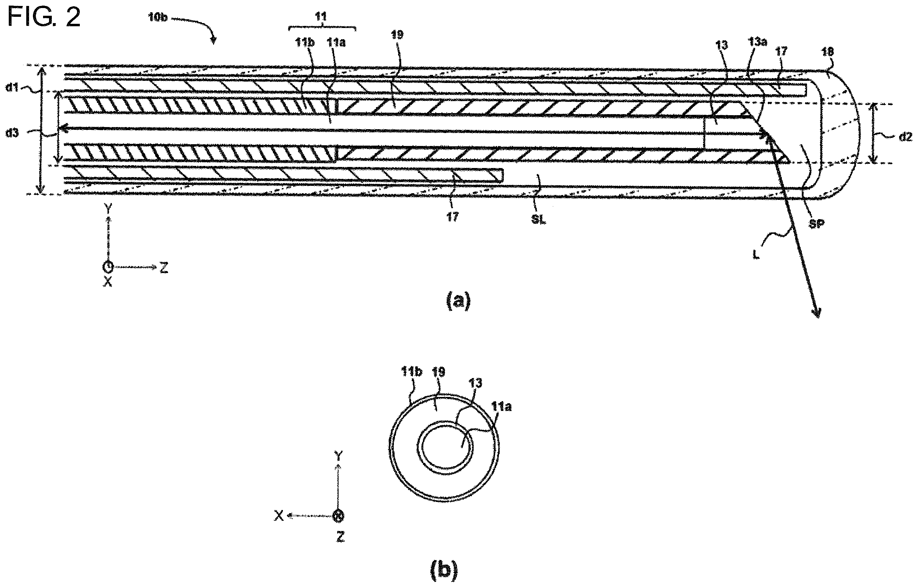

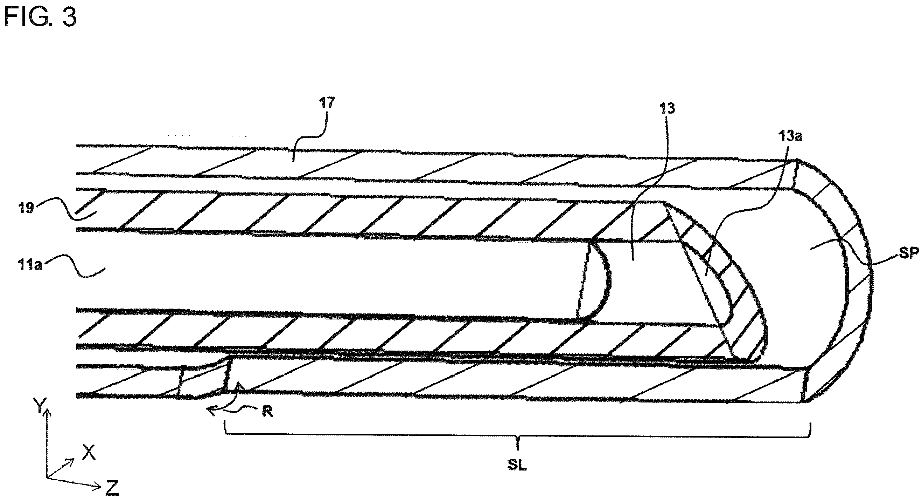

Figure 2 at a (a) region illustrates the YZ section of the distal end 10b of the optical probe 10. Figure 3 is a perspective view of the YZ section of the distal end 10b of the optical probe 10. Figure 2 at the (a) region and Figure 3 illustrate an XYZ rectangular coordinate system where a direction in which the optical fiber extends coincides with the Z-direction.

As illustrated in Figure 2 at the (a) region, the distal end 10b includes the optical fiber 11, a Grin lens 13 optically connected to the optical fiber 11, the metallic tube 17 that covers the optical fiber 11 and the Grin lens 13, and the needle 18 that is made of a resin and that covers the metallic tube 17. The optical fiber 11 and the Grin lens 13 are integrated by a molded portion 19. The needle 18 seals an interior space SP in an airtight manner. A medium that occupies the interior space SP may be a space, or a fluid may be filled therein. The outer diameter d1 of the needle 18 is 1 mm or less and is less than the outer diameter of the support tube 14. The metallic tube 17 has a slit SL formed in a manner in which a notch extending in the Z-direction from an end portion is made. This enables the rotational torque from the optical connector to be efficiently transmitted to the distal end via the support tube and the optical fiber. Accordingly, an optical probe that has an end having a reduced diameter and that is to be inserted into, for example, an organism is provided.

The optical fiber 11 is a single mode optical fiber and includes a glass fiber and a coating 11b that covers the glass fiber 11a. The glass fiber has a high-refractive-index core (not illustrated) through which light propagates and a low-refractive-index clad (not illustrated) that encompasses the core. At the end portion of the optical fiber 11 near the distal end 10b, a part of the coating 11b that has a predetermined length is removed, and the glass fiber 11a is exposed. The Grin lens 13 is connected to the end thereof by fusion bonding. The glass fiber 11a and the Grin lens 13 are encompassed by the molded portion 19. The optical fiber 11, the Grin lens 13, and the molded portion 19 are integrally formed. This enables the observation light deflected by the light deflection optical system to readily exit from the flank of the optical probe. In addition, this prevents portions of the optical fiber, the light collection optical system, and the light deflection optical system that are secured to each other from damaging due to rotation.

The diameters of the glass fiber 11a and the Grin lens 13 in the XY section perpendicular to an optical axis may be equal, or the diameter of the Grin lens may be slightly larger than the diameter of the glass fiber 11 a (about 1.02 to 1.10 times the diameter of the glass fiber 11 a). A difference in the diameter enables the interface between the Grin lens 13 and the glass fiber 11a to be readily recognized and enables the length of the Grin lens 13 to be readily managed.

The molded portion 19 is formed in a manner in which the glass fiber 11 a and the Grin lens 13 are fusion bonded, the optical fiber 11 is subsequently disposed inside molds, and a resin is filled therein and left to cure. The outer diameter of a portion of the molded portion 19 around the outer circumference of the glass fiber 11a is preferably equal to the outer diameter of a portion of the molded portion 19 around the outer circumference of the Grin lens 13. Thus, the molded portion 19 accommodates differences in the outer diameter between the glass fiber 11a and the Grin lens 13. Accordingly, the structure of the optical fiber 11, the Grin lens 13, and the molded portion 19 that are integrally formed is highly symmetric about the axis in the Z-direction. This enables a rotational torque to be efficiently transmitted to the distal end 10b in the case where the optical fiber is rotated about the axis in the Z-direction. The molded portion 19 may be formed of a resin through which the observation light L is transmissible, or the glass fiber 11 a and the Grin lens 13 may be inserted through a pipe member, such as a glass capillary, formed of a material through which the observation light L is transmissible and may be secured with an adhesive.

The outer diameter d2 of the molded portion 19 is preferably equal to or less than the diameter of the coating 11b so that the optical fiber 11 can be efficiently rotated inside the metallic tube 17. In the case where the optical fiber is a single mode optical fiber, the diameter of the glass fiber 11a is about 0.125 mm, the diameter of the coating 11b is about 0.25 mm, and the outer diameter d2 of the molded portion 19 is about 0.125 mm to 0.25 mm. In this case, the inner diameter d3 of the metallic tube 17 is preferably about 0.3 to 0.5 mm. The molded portion 19 is preferably formed of a resin, such as a fluorine resin, having a low coefficient of friction.

Figure 2 at the (b) region illustrates the front of the glass fiber 11a, the coating 11b, the molded portion 19, and the Grin lens 13 viewed from the distal end of the optical probe 10 in the Z-direction. The Grin lens that corresponds to a light collection optical system and a light deflection optical system is formed so as to be contained inside the section of the coating. The molded portion 19 is formed so as to be contained inside the section of the coating. Thus, the optical fiber 11, the molded portion 19, and the Grin lens 13 that rotate inside the distal end 10b are formed so as to be tapered as a whole. Accordingly, when a rotational torque is transmitted to the distal end 10b through the optical fiber 11, an end of the optical fiber 11 can be prevented from being displaced from a Z-axis, which is a rotational axis, and prevented from moving violently in the needle 18, and the rotational torque can be efficiently transmitted to the light deflection optical system.

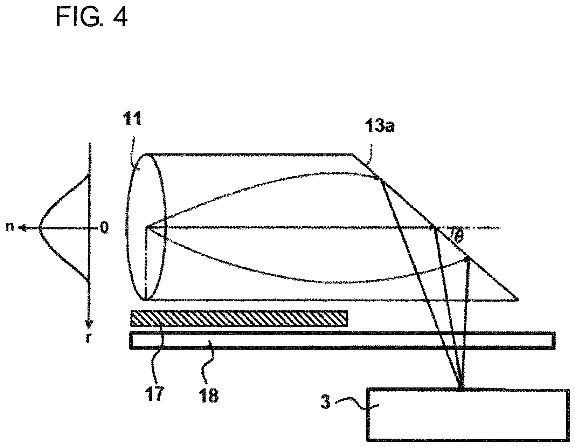

Figure 4 schematically illustrates the functions of the optical probe 10. At the distal end 10b, the edge surface of the Grin lens 13 includes a reflective surface 13a inclined to the Z-axis at an angle θ. A difference between the refractive index of the Grin lens 13 and the refractive index of the interior space SP enables light to be totally reflected and deflected. Accordingly, the Grin lens 13 has a function of serving as the light deflection optical system according to the present invention.

The Grin lens 13 also has a function of serving as the light collection optical system according to the present invention, collects light that has exited from the core of the optical fiber 11, and causes the light to exit therefrom. The Grin lens 13 has a refractive index distribution such that as a distance r from the optical axis extending in the Z-direction increases, the refractive index n gradually decreases, and the refractive index n is expressed as a quadratic function of the distance r. The refractive index of the Grin lens is rotationally symmetric about a central axis. Thus, light that has propagated in the fundamental mode of the optical fiber 11, exited from the core at the edge surface, and diverged converges while propagating substantially parallel to the Z-direction in the inside. During the convergence, the light is deflected by the reflective surface 13a. Thus, the light can be collected near a certain point at the outside.

According to the embodiment, although the Grin lens 13 that has the function of serving as the light collection optical system and the function of serving as the light deflection optical system is used, both the functions may be separated to different members. That is, the Grin lens 13 does not have the reflective surface 13a but has an edge surface perpendicular to the Z-axis, and has only the function of serving as the light collection optical system. A member having the function of serving as the light deflection optical system, such as a prism having the reflective surface 13a, may be secured to the edge surface.

The metallic tube 17 has the slit SL formed in a manner in which the notch extending in the Z-direction from the end portion is made. The needle 18 and the molded portion 19 are formed of a material through which the observation light L propagating through the optical fiber 11 is transmissible. Thus, the observation light L propagating through the optical fiber 11 is deflected in the Y-direction by the reflective surface 13a while being collected by the Grin lens 13, subsequently passes through the interior space SP, the slit SL, and the needle 18, and is incident on the object 3 located on the flank of the distal end 10b. The angle θ formed between the reflective surface 13a and the central axis is preferably determined to be no less than 20° and less than 45° such that the observation light L exits oblique to the Z-direction with respect to the Y-direction.

In the optical probe 10, the optical fiber 11 and the support tube 14 can rotate about the axis inside the jacket tube 15 while the optical connector 12 rotates. The rotational torque of the support tube 14 and the optical fiber 11 is transmitted to the optical fiber 11 inside the distal end 10b through the optical fiber 11 held in the through-hole of the handpiece 16. Accordingly, the optical fiber 11 and the Grin lens 13 can be rotated about the Z-axis as the rotational axis inside the metallic tube 17 and the needle 18 at the distal end 10b in a manner in which the optical connector 12 is rotated.

In the optical probe 10, the optical fiber 11 can rotate about the axis inside the metallic tube 17 having the slit SL. This enables the distal end to be suitably prevented from moving violently from the rotational axis toward the outside due to deformation of the needle 18 when an external force is applied to the needle 18 via the handpiece 16. The observation light that is scanned over the object on the flank of the optical probe 10 is limited by an opening range R about the Z-axis of the slit SL. This inhibits a region of the object 3 other than the region to be observed from being irradiated with the observation light L and prevents an unexpected portion from damaging.

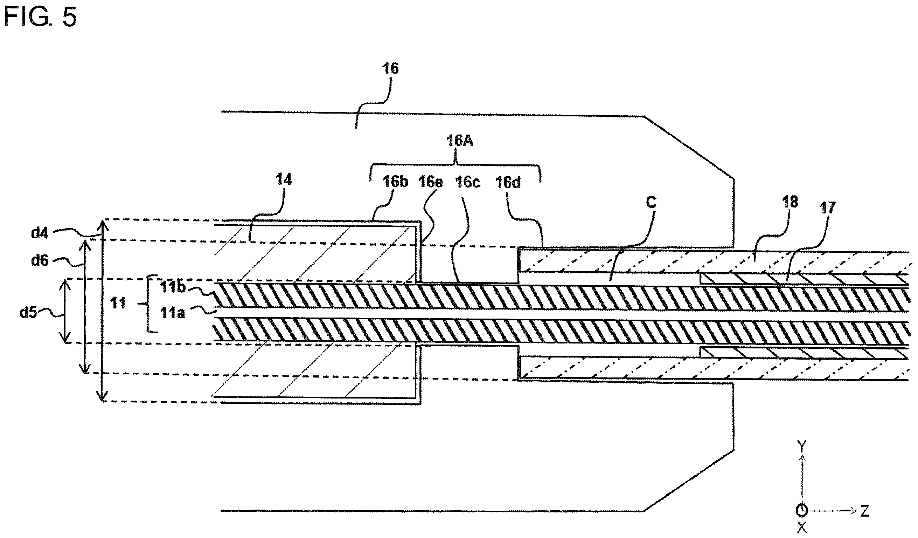

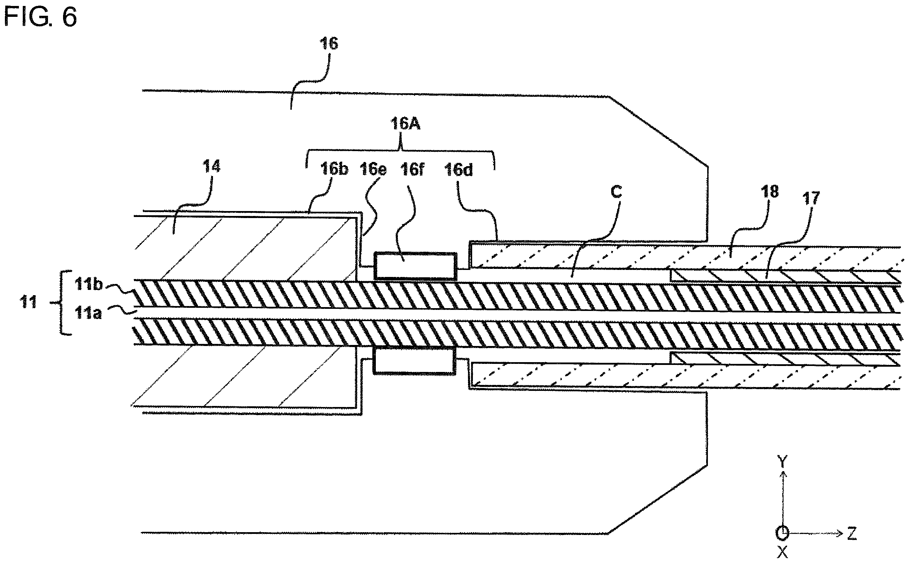

Figure 5 and Figure 6 illustrate the YZ section of the handpiece 16 on the side of the distal end 10b. The handpiece 16 holds the optical fiber 11 such that the optical fiber 11 is rotatable inside the distal end 10b. The through-hole 16A of the handpiece 16 includes the second section 16b, the third section 16c, and the third section 16d, as described above.

The outer surface of the metallic tube 17 is secured to the inner surface of the needle 18 with an adhesive. The outer surface of the needle 18 is secured to the inner surface of the fourth section 16d with an adhesive. The inner diameter d2 of the metallic tube 17 is slightly larger than the outer diameter of the optical fiber 11. Thus, the fourth section 16d rotatably accommodates the optical fiber 11 inside the metallic tube 17 and the needle 18.

The second section 16b rotatably accommodates the optical fiber 11 and the support tube 14. A contact surface 16e is formed between the second section 16b and the third section 16c to position the support tube 14. The third section 16c rotationally accommodates the optical fiber 11. Accordingly, the handpiece 16 accommodates the optical fiber 11 inside the through-hole 16A such that the optical fiber 11 is rotatable inside the distal end 10b.

The rotational torque applied by the optical connector 14 is transmitted nearer than the handpiece 16 to the proximal end 10a by using the support tube 14 and the optical fiber 11 and is transmitted nearer than the handpiece 16 to the distal end 10b by using only the optical fiber. Since there is a boundary portion thereof inside the handpiece 16, the rotational torque can be efficiently transmitted to the distal end 10b in a manner in which a clearance between the through-hole 16A and the support tube 14 and between the through-hole 16A and the optical fiber 11 is decreased.

The needle 18 accommodates the optical fiber 11, and the outer diameter d1 thereof (and the inner diameter d3 of the metallic tube 17) is larger than the outer diameter of the optical fiber 11. According to the embodiment of the present invention, the outer diameter d1 of the needle 18 is less than the outer diameter of the support tube 14. Accordingly, the inner diameter d4 of the second section 16b, the inner diameter d5 of the third section 10c, and the inner diameter d6 of the fourth section 10d, within which the support tube 14, the optical fiber 11, and the needle 18 are respectively accommodated, preferably satisfy the relationship of d4 > d6 > d5. This enables a clearance between the handpiece and the support tube, between the handpiece and the optical fiber, and between the handpiece and the needle to be decreased, and enables the rotational torque to be efficiently transmitted to the distal end even when the handpiece is interposed.

In the case where an adhesive enters the inner surface of the metallic tube 17 when the outer surface of the needle 18 is stuck to the inner surface of the fourth section 16d, there is a risk of impeding rotation of the optical fiber 11. Accordingly, a clearance C is preferably left between the edge surface of the metallic tube 17 on the side of the proximal end 10a and the third section 16c. In this case, the edge surface of the metallic tube 17 on the side of the proximal end 10a is located nearer than the edge surface of the needle 18 on the side of the proximal end 10a to the distal end 10b. This enables rotation of the optical fiber to be prevented from being impeded by an adhesive inside the handpiece and enables the rotational torque to be efficiently transmitted to the distal end. In addition, the area of the needle 18 stuck to the fourth section 16d can be sufficiently ensured while the clearance C is maintained.

The coefficient of friction of the inner wall of the third section 16c, which can be come in contact with the coating 11b of the optical fiber 11, is preferably low so that the rotational torque of the optical connector 12 is efficiently transmitted to a location, which is not held by the support tube 14, nearer than the third section 16c to the distal end 10b. Accordingly, as illustrated in Figure 6, the third section 16c may be defined by the inner surface of an O-ring 16f formed of, for example, a fluorine resin. The inner diameter of the O-ring 16f corresponds to the inner diameter d5 of the third section 16c. This prevents the optical fiber from damaging during rotation at a location at which the optical fiber is exposed inside the handpiece 16. In this case, it is preferable that the O-ring 16f be disposed in a manner where the contact surface 16e for positioning of the support tube 14 be formed adjacent to the third section 16c on the side of the proximal end 10a.

Similarly, the coefficient of friction of the inner surface of the metallic tube 11, in which the optical fiber 11 rotates, is preferably low. The outer wall of the metallic tube 11 is secured to the inner wall of the needle 18 with an adhesive, and the coefficient of friction of the outer surface of the metallic tube 17 is preferably high. Accordingly, surface roughness of the inner surface of the metallic tube 17 is preferably less than surface roughness of the outer surface thereof. Thus, an anchor effect on the rough outer surface of the metallic tube 17 enables the metallic tube 17 to be firmly secured to the needle 18, and rotation of the optical fiber 11 and the Grin lens 13, which are encompassed by the inner surface of the metallic tube 17 that has a low coefficient of friction, are prevented from impeded.

### Modification

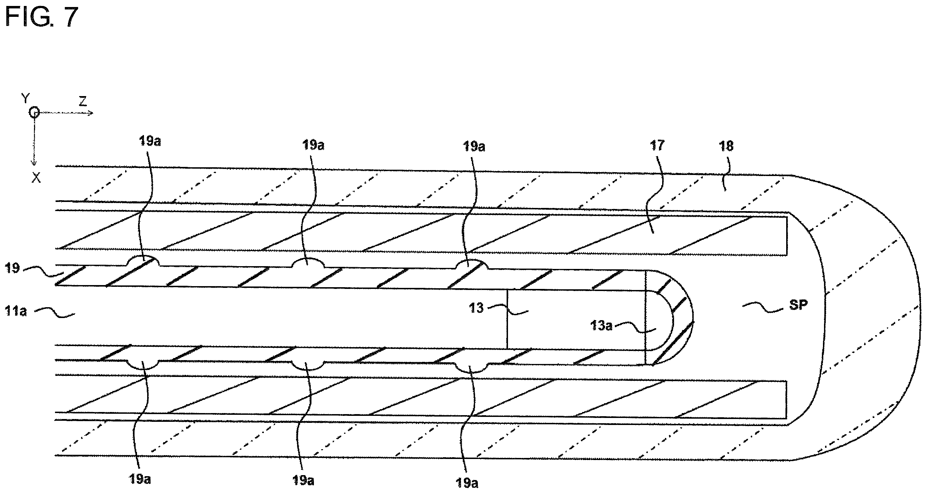

Figure 7 illustrates the XY section of the distal end 10b of an optical probe according to a modification to the embodiment. The modification differs from the above embodiment in that the molded portion 19 includes protrusions 19a. The protrusions 19a are formed on parts of the molded portion 19 extending in the Z-direction so as to protrude from the outer circumference toward the needle 18. It is preferable that the protrusions 19a be integrally formed with the molded portion 19 by using the same resin when the molded portion 19 is disposed around the outer circumference of the Grin lens 13 and the optical fiber 11.

The protrusions 19a enable the optical fiber 11 to be located at the central position of the needle 18. That is, in the case where the optical fiber 11 is rotated about the Z-axis as the rotational axis, the protrusions 19a can come in contact with the inner circumference of the needle 18 (inner circumference of the metallic tube 17) and restrict movement of the optical fiber 11 even when the optical fiber 11 violently moves in the needle 18. Accordingly, the rotational torque can be efficiently transmitted to the light deflection optical system.

Some of the protrusions 19a are preferably arranged in a section perpendicular to the axis of the molded portion 19. For example, four protrusions are arranged in the circumferential direction at an interval of 90°, or three protrusions are arranged in the circumferential direction at an interval of 120°. Some of the protrusions 19a are preferably arranged in the Z-direction. In this case, the optical fiber 11 and the Grin lens 13 can be readily located at the central position of the cylindrical needle 18 in a manner in which the heights of the protrusions 19a are equal. It is preferable that the height of each protrusion 19a be determined such that the top thereof is higher than the coating 11b and there is a small clearance between the inner surface of the metallic tube 17 and the protrusion 19a.

## Claims

1. An optical probe, comprising:
an optical fiber configured to transmit observation light between a proximal end to be connected to a measurement unit of an OCT device and a distal end configured to exit the observation light;
an optical connector that is connected to the optical fiber at the proximal end and that is configured to connected to the measurement unit;
a light collection optical system that is optically connected to the optical fiber at the distal end and that is configured to collect the observation light that exits from the optical fiber;
a light deflection optical system that is optically connected to the light collection optical system at the distal end and that is configured to deflect the observation light that exits from the optical fiber;
a needle that is formed of a material through which the observation light is transmissible and that rotatably accommodates the optical fiber, the light collection optical system, and the light deflection optical system at the distal end;
a handpiece that has a through-hole, that rotatably accommodates a part of the optical fiber inside the through-hole between the proximal end and the distal end, and that holds the needle; and
a support tube that is secured to the optical connector on a proximal end side and that is rotatably accommodated in the handpiece on a distal end side,
wherein an outer diameter of the needle is less than an outer diameter of the support tube.

2. The optical probe according to Claim 1,
wherein the through-hole includes a second section that rotatably accommodates the optical fiber and the support tube, a third section that rotatably accommodates the optical fiber, and a fourth section that secures the needle and that rotatably accommodates the optical fiber, and
wherein a relationship of
an inner diameter of the second section > an inner diameter of the fourth section
> an inner diameter of the third section
holds.

3. The optical probe according to Claim 2,
wherein the third section is formed of a material having a coefficient of friction less than a coefficient of friction of the second section and the fourth section.

4. The optical probe according to any one of Claims 1 to 3, further comprising:
a metallic tube that is secured inside the needle and that rotatably accommodates the optical fiber, the light collection optical system, and the light deflection optical system.

5. The optical probe according to Claim 4,
wherein the metallic tube has, at a location in a circumferential direction, a slit that is configured to cause the observation light deflected by the light deflection optical system to exit from the distal end.

6. The optical probe according to Claim 4 or Claim 5,
wherein surface roughness of an inner surface of the metallic tube that rotatably accommodates the optical fiber, the light collection optical system, and the light deflection optical system is less than surface roughness of an outer surface thereof that is a surface secured inside the needle.

7. The optical probe according to any one of Claims 4 to 6,
wherein the needle is secured to the fourth section with an adhesive, and
wherein an end portion of the metallic tube on the proximal end side is located nearer than an end portion of the needle on the proximal end side to the distal end.

8. The optical probe according to any one of Claims 1 to 7,
wherein the optical fiber includes, in the needle, a coating and a glass fiber around which the coating is removed, and
wherein an outer circumference of the light deflection optical system, the light collection optical system, and the glass fiber is covered by a molded portion through which the observation light is transmissible.

9. The optical probe according to Claim 8, wherein an outer diameter of the molded portion is less than an outer diameter of the coating when viewed in a direction of an optical axis of the optical fiber.

10. The optical probe according to Claim 8 or Claim 9,
wherein the molded portion includes, on a part extending in an optical axis of the optical fiber, a protrusion that protrudes from an outer circumference of the molded portion toward the needle.

11. The optical probe according to Claim 10,
wherein the protrusion comprises a plurality of protrusions that are arranged in a circumferential direction of the optical fiber and that are equal in height from the molded portion.
